# EUROPÄISCHE PATENTSCHRIFT

(11) **EP 3 275 858 B1**
(45) Veröffentlichungstag und Bekanntmachung des Hinweises auf die Patenterteilung: **14.11.2018**
(21) Anmeldenummer: 16181360.5
(22) Anmeldetag: 27.07.2016
(51) Int. Cl.: C07C 209/84, C07C 211/36

(54) **ABTRENNUNG VON LEICHTSIEDERN SOWIE REDUKTION DES AMMONIAKGEHALTES IM ISOPHORONDIAMIN DURCH PARTIALKONDENSATION**
SEPARATION OF LIGHT VOLATILES AND REDUCTION OF THE AMMONIA CONTENT IN ISOPHORONDIAMINE BY MEANS OF PARTIAL CONDENSATION
SEPARATION DE PRODUITS A POINT BAS D'EBULLITION ET REDUCTION DE LA TENEUR EN AMMONIAC DANS D'ISOPHORONEDIAMINE PAR CONDENSATION PARTIELLE

(43) Veröffentlichungstag der Anmeldung: 31.01.2018
(73) Patentinhaber: Evonik Degussa GmbH, 45128 Essen (DE)
(72) Erfinder: RITTSTEIGER, Anne, 59399 Olfen (DE); CASSENS, Jan, 45665 Recklinghausen (DE); HENGSTERMANN, Axel, 48308 Senden (DE); KNOOP, Cord, 15721 Haltern am See (DE); MÜLLER, Anja, 44135 Dortmund (DE); RÜFER, Alexander Martin, 45657 Recklinghausen (DE)

(56) Entgegenhaltungen:
- EP-B1- 1 529 027

## Beschreibung

Die Erfindung betrifft die Feinreinigung von Isophorondiamin (IPDA) mit Hilfe eines zweistufigen Kolonnenaufbaus mit Partialkondensator und Rückführung.
Die Herstellung von IPDA durch aminierende Hydrierung von Isophoronnitril (IPN) ist bekannt und wurde bereits mehrfach beschrieben.

Im einfachsten Fall (US 3,352,913) wird IPN in Gegenwart von Wasserstoff und eines Überschusses an Ammoniak an einem Cobaltkatalysator zur Reaktion gebracht. Zunächst bildet sich aus IPN und Ammoniak durch Wasserabspaltung das Isophoronnitrilimin, IPNI, das nachfolgend zum IPDA hydriert wird:

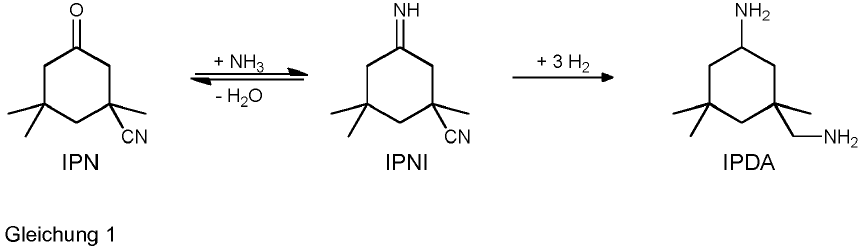

Weiterhin sind Verfahren zur Herstellung von Isophorondiamin aus CN 104230721A, EP 2 649 042A und WO 2012126869A bekannt.

Isophorondiamin wird gemäß der EP 2 649 042A in einer ein- oder zweistufigen Reaktion aus Isophoronnitril hergestellt. Dabei wird Isophoronnitril zunächst mit Ammoniak zu Isophoronnitrilimin iminiert. Im zweiten Schritt wird dieses zu Isophorondiamin hydriert. Die an die Reaktion angeschlossene Aufreinigung gliedert sich ebenfalls in zwei Schritte. Zunächst werden in mehreren Destillationskolonnen die Leichtsieder abgetrennt, dazu gehören Wasserstoff, Inertgase, Ammoniak und leichtsiedende Verunreinigungen (Leichtsiederabtrennung). In einem letzten Schritt wird dann das Rein-Isophorondiamin durch zwei Vakuumdestillationskolonnen gewonnen. Die erste Kolonne dient wiederum der Abtrennung von noch enthaltenen leichter siedenden Nebenprodukten. In der zweiten Kolonne wird das Isophorondiamin rein über Kopf gewonnen und so von den organischen Rückständen (Schwersieder) getrennt.

In der WO 2015/038679 wird ein Verfahren zur Trennung von Ammoniak und einem Diamin beschrieben. Hierbei erfolgt die Destillation über eine Folge aus drei Destillationskolonnen, wobei in jeder Kolonne Ammoniak als Leichtsieder über Kopf abgetrennt wird.

In den beiden Anmeldungen EP 1529027 und EP 1529028 wird die Feinreinigung von IPDA durch Destillation in einem Aufbau aus mindestens zwei Kolonnen beschrieben. Hierbei erfolgt sowohl die Abtrennung von leicht- und schwersiedenden Nebenkomponenten, als auch die Auftrennung in zwei separate IPDA-Fraktionen. Diese unterscheiden sich jeweils in ihrem cis/trans-Verhältnis.

Aufgabe der vorliegenden Erfindung ist es, ein einfaches Verfahren zur Feinreinigung von Isophorondiamin zu finden mit reduziertem Ammoniak-Gehalt im Rein-Isophorondiamin.

Überraschenderweise wurde gefunden, dass sich durch den Einsatz eines zusätzlichen Partialkondensators am Kopf der zweiten Kolonne eines Zweikolonnensystems zur Feindestillation von Roh-IPDA mit Rückführung eines Teilstroms in die erste Kolonne der Ammoniak-Gehalt im Rein-IPDA reduzieren lässt.

Gegenstand der Erfindung ist ein Verfahren zur Feinreinigung von Isophorondiamin aus der Herstellung von Isophorondiamin durch aminierende Hydrierung von Isophoronnitril in Anwesenheit von mindestens Ammoniak, Wasserstoff, eines Hydrierkatalysator und gegebenenfalls weiteren Zusätzen und in An- oder Abwesenheit von organischen Lösungsmitteln, wobei ein Roh-Isophorondiamin I erhalten wird,
dadurch gekennzeichnet, dass das Roh-Isophorondiamin I durch zwei Vakuumdestillationskolonnen einer Feinreinigung unterworfen wird, wobei
I. in der ersten Vakuumdestillationskolonne K I die Abtrennung von noch enthaltenen leichter siedenden Nebenprodukten erfolgt, und ein Roh-IPDA II aus dem Sumpf von K I in die Vakuumdestillationskolonne K II überführt wird,
II. und in der zweiten Vakuumdestillationskolonne K II das Isophorondiamin rein über Kopf gewonnen und von den organischen Rückständen getrennt wird, wobei am Kopf der Vakuumdestillationskolonne K II zwei Kondensatoren angebracht sind,
   wobei der erste Kondensator ein Partialkondensator ist und in dem das Rein-IPDA abgetrennt wird,
   und wobei der zweite Kondensator ein Totalkondensator ist und in dem der restliche Teil des Dampfstroms aus K II komplett kondensiert und in die erste Vakuumdestillationskolonne K I als Rückstrom zurückgeführt wird.

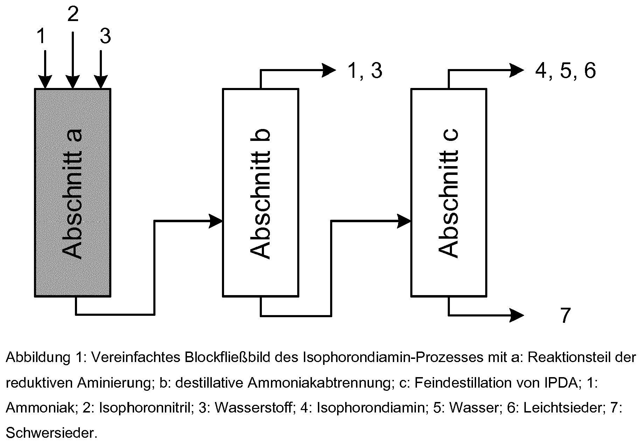

Der gesamte Prozess zur Herstellung von Rein-IPDA gliedert sich in drei Abschnitte (siehe Abbildung 1). In Abschnitt a erfolgt die Reaktion durch aminierende Hydrierung von Isophoronnitril in einem ein- oder mehrstufigen Prozess unter Anwesenheit von mindestens Ammoniak, Wasserstoff und einem Katalysator. In Abschnitt b erfolgt die destillative Abtrennung von Ammoniak und Wasserstoff zur Gewinnung von Roh-IPDA. Die Destillation kann in einer oder mehreren Kolonnen durchgeführt werden. In Abschnitt c erfolgt die Feinreinigung vom Roh-IPDA durch destillative Abtrennung von IPDA, Wasser, Leichtsieder und Schwersieder. Die Feinreinigung wird in zwei Vakuumdestillationskolonnen durchgeführt.

Das eingesetzte Roh-IPDA I weißt im Allgemeinen die folgende Zusammensetzung in Gewichts-% (Gew.-%) auf:

| | |
|---|---|
| IPDA | 75-100 Gew.-% |
| Wasser | 0-15 Gew.-% |
| Leichtsieder | 0-6 Gew.-% |
| Schwersieder | 0-6 Gew.-% |
| Rest-Ammoniak | 10-1000 ppm |

Leichtsieder sind dabei so definiert, dass es sich hierbei um Nebenprodukte aus dem Herstellungsprozess von IPDA handelt mit einem niedrigeren Siedepunkt als IPDA. Schwersieder sind dabei so definiert, dass es sich hierbei um Nebenprodukte aus dem Herstellungsprozess von IPDA handelt mit einem höheren Siedepunkt als IPDA.

Das erfindungsgemäße Verfahren ist im Allgemeinen dadurch gekennzeichnet, dass das Roh-Isophorondiamin I durch zwei Vakuumdestillationskolonnen einer Feinreinigung unterworfen wird,
wobei
I. in der ersten Vakuumdestillationskolonne K I die Abtrennung von noch enthaltenen leichter siedenden Nebenprodukten erfolgt, und ein Roh-IPDA II aus dem Sumpf von K I in die Vakuumdestillationskolonne K II überführt wird,
II. und in der zweiten Vakuumdestillationskolonne K II das Isophorondiamin rein über Kopf gewonnen und von den organischen Rückständen getrennt wird, wobei am Kopf der Vakuumdestillationskolonne K II zwei Kondensatoren angebracht sind,
   wobei der erste Kondensator ein Partialkondensator ist und in dem das Rein-IPDA abgetrennt wird,
   und wobei der zweite Kondensator ein Totalkondensator ist und in dem der restliche Teil des Dampfstroms aus K II komplett kondensiert und in die erste Vakuumdestillationskolonne K I als Rückstrom zurückgeführt wird.

Die erste eingesetzte Vakuumdestillationskolonne K I weist folgende Parameter auf:

| | |
|---|---|
| Temperatur | 40-120°C |
| Druck | 10-200 mbar |
| Theoretische Stufen | 10-80 |

Die Zusammensetzung des Zustroms Roh-IPDA II aus der ersten Vakuumdestillationskolonne in die zweite Vakuumdestillationskolonne weist folgende Zusammensetzung auf:

| | |
|---|---|
| IPDA | 90-100 Gew.-% |
| Schwersieder | 0-10 Gew.-% |
| Rest-Ammoniak | 10-500 ppm |

Die eingesetzte zweite Vakuumdestillationskolonne K II weist folgende Parameter auf:

| | |
|---|---|
| Druck | 10-200 mbar |
| Kopftemperatur | 80-200°C |
| Theoretische Stufen | 5-50 |

Der eingesetzte Partialkondensator am Kopf der zweiten Vakuumdestillationskolonne weist folgende Parameter auf:

| | |
|---|---|
| Druck | 10-200 mbar |
| Temperatur | 80-200°C |

Die Reinheit des Rein-Isophorondiamins beträgt mindestens 98 Gew.-%. Der Rest-Ammoniak-Gehalt im Rein-IPDA beträgt kleiner 50 ppm.

### Bevorzugte Verfahrensweisen und Verfahrensschema

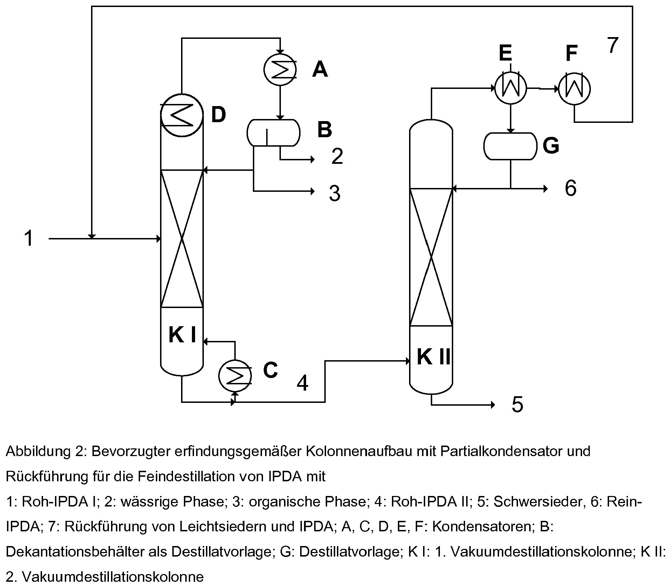

Das Roh-IPDA I wird zunächst in die erste Vakuumdestillationskolonne K I geleitet, siehe Abbildung 2.

Die Leichtsieder (Strom 2) und Wasser (Strom 3) werden dabei über Kopf der ersten Vakuumdestillationskolonne abgetrennt. Das IPDA verlässt die Kolonne als Sumpfstrom 4 (Roh-IPDA II) und wird der zweiten Vakuumdestillationskolonne K II zur Abtrennung der Schwersieder (Strom 5) und der Gewinnung von Rein-IPDA (Strom 6) zugeführt. Am Kopf der zweiten Vakuumdestillationskolonne sind zwei in Reihe geschaltete Kondensatoren E und F angebracht. Der erste Kondensator E wird dabei als Partialkondensator betrieben. Hierbei wird ein Teil des Dampfstroms aus dem Kopf der zweiten Vakuumdestillationskolonne kondensiert und dadurch das Rein-IPDA als Strom 6 gewonnen. Der restliche Teil des Dampfstroms wird im zweiten Kondensator F komplett kondensiert und in der ersten Vakuumdestillationskolonne K II als Rückstrom 7 zurückgeführt. Dadurch wird auch der Großteil des Ammoniak-Gehaltes aus Roh-IPDA II über die Kondensatoren von IPDA getrennt und über Strom 7 in die Vakuumdestillationskolonne K I rückgeführt. Dies ermöglicht die Gewinnung von Rein-IPDA mit einer sehr hohen Qualität und sehr geringem Ammoniak Gehalt.

### Beispiele

### Beispiel 1

Die Simulation der Destillation erfolgte über Aspen Plus. Für die Berechnungen wurde ein Destillationsaufbau bestehend aus zwei Vakuumdestillationskolonnen betrachtet.

Der eingesetzte Feedstrom (Roh-IPDA I) hatte folgende Zusammensetzung in Gewichts-% (Gew.-%):

| | |
|---|---|
| IPDA | 85,8 Gew.-% |
| Wasser | 9,7 Gew.-% |
| Schwersieder | 2,3 Gew.-% |
| Leichtsieder und Rest-NH₃ | 2,2 Gew.-% |

In der ersten Vakuumdestillationskolonne mit 42 Trennstufen wurden die organischen Leichtsieder und Wasser aus dem eintretenden Roh-IPDA I Strom als Destillat über Kopf abgetrennt und im Anschluss die organische und die wässrige Phase nach der Kondensation in einem Dekanter getrennt. Ein Teil der organischen Phase wurde als Rücklauf in die erste Vakuumdestillationskolonne mit einem Rücklauf-zu-Feed-Verhältnissen von 1,2 gegeben. Die Vakuumdestillationskolonne wurde bei 110 mbar, einer Sumpftemperatur von 178°C und einer Kopftemperatur von 114°C betrieben.

Der Feedstrom von der ersten in die zweite Vakuumdestillationskolonne hatte folgende Zusammensetzung (Roh-IPDA II):

| | |
|---|---|
| IPDA | 97,4 Gew.-% |
| Schwersieder | 2,6 Gew.-% |
| Rest-Ammoniak | 247 ppm |

Die zweite Vakuumdestillationskolonne mit 13 theoretischen Trennstufen wurde bei einer Sumpftemperatur von 207°C, einer Kopftemperatur von 168°C und einem Druck von 110 mbar betrieben. Die Schwersieder wurden dabei über den Sumpf der Vakuumdestillationskolonne abgetrennt, IPDA und das restliche Ammoniak über Kopf. Das Kopfprodukt wurde als Dampfphase in einen Partialkondensator geleitet und bei 165°C und 110 mbar zum Teil auskondensiert. Das Massenverhältnis aus Partialkondensat und nicht-kondensierter Dampfphase lag bei 130. Der nicht-kondensierte Dampfstrom wurde im Anschluss in einem zweiten Kondensator bei 133°C und 110 mbar vollständig kondensiert und als Rückstrom in die erste Vakuumdestillationskolonne geleitet. Aus dem Kondensat des Partialkondensators wurde Rein-IPDA mit einer Reinheit von 99,9 Gew.-% und einem Rest-Ammoniak-Gehalt von 15 ppm gewonnen.

Durch den Einsatz eines Partialkondensators am Kopf der zweiten Vakuumdestillationskolonne konnte der Rest-Ammoniak-Gehalt somit von 247 ppm im Roh-IPDA auf 15 ppm im Rein-IPDA reduziert werden.

### Beispiel 2

Die Simulation der Destillation erfolgte über Aspen Plus. Für die Berechnungen wurde ein Destillationsaufbau bestehend aus zwei Vakuumdestillationskolonnen betrachtet.

Der eingesetzte Feedstrom (Roh-IPDA I) hatte folgende Zusammensetzung in Gewichts-% (Gew.-%):

| | |
|---|---|
| IPDA | 85,8 Gew.-% |
| Wasser | 9,7 Gew.-% |
| Schwersieder | 2,3 Gew.-% |
| Leichtsieder und Rest-NH₃ | 2,2 Gew.-% |

In der ersten Vakuumdestillationskolonne mit 42 Trennstufen wurden die organischen Leichtsieder und Wasser aus dem eintretenden Roh-IPDA I Strom als Destillat über Kopf abgetrennt und im Anschluss die organische und die wässrige Phase nach der Kondensation in einem Dekanter getrennt. Ein Teil der organischen Phase wurde als Rücklauf in die erste Vakuumdestillationskolonne mit einem Rücklauf-zu-Feed-Verhältnissen von 1,2 gegeben. Die Vakuumdestillationskolonne wurde bei 110 mbar, einer Sumpftemperatur von 178°C und einer Kopftemperatur von 114°C betrieben.

Der Feedstrom von der ersten in die zweite Vakuumdestillationskolonne hatte folgende Zusammensetzung (Roh-IPDA II):

| | |
|---|---|
| IPDA | 97,4 Gew.-% |
| Schwersieder | 2,6 Gew.-% |
| Rest-Ammoniak | 104 ppm |

Die zweite Vakuumdestillationskolonne mit 13 theoretischen Trennstufen wurde bei einer Sumpftemperatur von 207°C, einer Kopftemperatur von 168°C und einem Druck von 110 mbar betrieben. Die Schwersieder wurden dabei über den Sumpf der Kolonne abgetrennt, IPDA und das restliche Ammoniak über Kopf. Das Kopfprodukt wurde als Dampfphase in einen Partialkondensator geleitet und bei 165°C und 110 mbar zum Teil auskondensiert. Das Massenverhältnis aus Partialkondensat und nicht-kondensierter Dampfphase lag bei 344. Der nicht-kondensierte Dampfstrom wurde im Anschluss in einem zweiten Kondensator bei 133°C und 110 mbar vollkondensiert und als Rückstrom in die erste Vakuumdestillationskolonne geleitet.

Aus dem Kondensat des Partialkondensators wurde Rein-IPDA mit einer Reinheit von 99,9 Gew.-% und einem Rest-Ammoniak-Gehalt von 15 ppm gewonnen.

Durch den Einsatz eines Partialkondensators am Kopf der zweiten Vakuumdestillationskolonne konnte der Rest-Ammoniak-Gehalt von 104 ppm im Roh-IPDA auf 15 ppm im Rein-IPDA reduziert werden.

### Beispiel 3: Vergleichsbeispiel

Die Simulation der Destillation erfolgte über Aspen Plus. Für die Berechnungen wurde die Destillation bestehend aus zwei Vakuumdestillationskolonnen betrachtet. Im Vergleich zu den erfindungsgemäßen Beispielen wurde in diesem Fall lediglich ein Totalkondensator am Kopf der zweiten Vakuumdestillationskolonne betrachtet.

Der eingesetzte Feedstrom (Roh-IPDA I) hatte folgende Zusammensetzung in Gewichts-% (Gew.-%):

| | |
|---|---|
| IPDA | 85,8 Gew.-% |
| Wasser | 9,7 Gew.-% |
| Schwersieder | 2,3 Gew.-% |
| Leichtsieder und Rest-NH₃ | 2,2 Gew.-% |

In der ersten Vakuumdestillationskolonne mit 42 Trennstufen wurden die organischen Leichtsieder und Wasser aus dem eintretenden Roh-IPDA I Strom als Destillat über Kopf abgetrennt und im Anschluss die organische und die wässrige Phase nach der Kondensation in einem Dekanter getrennt. Ein Teil der organischen Phase wurde als Rücklauf in die erste Vakuumdestillationskolonne mit einem Rücklauf-zu-Feed-Verhältnissen von 1,2 gegeben. Die Vakuumdestillationskolonne wurde bei 110 mbar, einer Sumpftemperatur von 178°C und einer Kopftemperatur von 114°C betrieben.

Der Feedstrom von der ersten in die zweite Vakuumdestillationskolonne hatte folgende Zusammensetzung (Roh-IPDA II):

| | |
|---|---|
| IPDA | 97,4 Gew.-% |
| Schwersieder | 2,6 Gew.-% |
| Rest-Ammoniak | 50 ppm |

Die zweite Vakuumdestillationskolonne mit 13 theoretischen Trennstufen wurde bei einer Sumpftemperatur von 207°C, einer Kopftemperatur von 168°C und einem Druck von 110 mbar betrieben. Die Schwersieder wurden dabei über den Sumpf der Kolonne abgetrennt, IPDA und das restliche Ammoniak über Kopf. Das Kopfprodukt wurde bei 133°C vollständig auskondensiert.

Aus dem Kondensat wurde Rein-IPDA mit einer Reinheit von 99,9 Gew.-% und einem Rest-Ammoniak-Gehalt von 50 ppm gewonnen. Der Rest-Ammoniak-Gehalt im Rein-IPDA konnte somit im Vergleich zum Roh-IPDA nicht reduziert werden.

Aus den aufgeführten Beispielen geht hervor, dass die Anwendung der Partialkondensation deutlich zur Verringerung des Rest-Ammoniak Gehaltes im Rein-IPDA führt. Zudem wurde gezeigt, dass durch die Verwendung des Partialkondensators nach der zweiten Vakuumdestillationskolonne auch bei variierenden Rest-Ammoniak Gehalten im Roh-IPDA II Strom der Ammoniakgehalt im Rein-IPDA deutlich unterhalb von 50 ppm lag.

## Patentansprüche

1. Verfahren zur Feinreinigung von Isophorondiamin aus der Herstellung von Isophorondiamin durch aminierende Hydrierung von Isophoronnitril in Anwesenheit von mindestens Ammoniak, Wasserstoff, eines Hydrierkatalysator und gegebenenfalls weiteren Zusätzen und in An- oder Abwesenheit von organischen Lösungsmitteln, wobei ein Roh-Isophorondiamin I erhalten wird,
**dadurch gekennzeichnet, dass** das Roh-Isophorondiamin I durch zwei Vakuumdestillationskolonnen einer Feinreinigung unterworfen wird, wobei
I. in der ersten Vakuumdestillationskolonne K I die Abtrennung von noch enthaltenen leichter siedenden Nebenprodukten erfolgt, und ein Roh-IPDA II aus dem Sumpf von K I in die Vakuumdestillationskolonne K II überführt wird,
II. und in der zweiten Vakuumdestillationskolonne K II das Isophorondiamin rein über Kopf gewonnen und von den organischen Rückständen getrennt wird, wobei am Kopf der Vakuumdestillationskolonne zwei Kondensatoren angebracht sind,
wobei der erste Kondensator ein Partialkondensator ist und in dem das Rein-IPDA abgetrennt wird,
und wobei der zweite Kondensator ein Totalkondensator ist und in dem der restliche Teil des Dampfstroms aus K II komplett kondensiert und in die erste Vakuumdestillationskolonne K I als Rückstrom zurückgeführt wird.

2. Verfahren nach Anspruch 1, **dadurch gekennzeichnet, dass** das Roh-Isophorondiamin I im Allgemeinen die folgende Zusammensetzung aufweist:
| | |
|---|---|
| IPDA | 75-100 Gew.-% |
| Wasser | 0-15 Gew.-% |
| Leichtsieder | 0-6 Gew.-% |
| Schwersieder | 0-6 Gew.-% |
| Rest-Ammoniak | 10-1000 ppm. |

3. Verfahren nach mindestens einem der vorherigen Ansprüche, **dadurch gekennzeichnet, dass** die erste Vakuumdestillationskolonne K I die folgenden Parameter aufweist:
| | |
|---|---|
| Temperatur | 40-120°C |
| Druck | 10-200 mbar |
| Theoretische Stufen | 10-80. |

4. Verfahren nach mindestens einem der vorherigen Ansprüche, **dadurch gekennzeichnet, dass** die Zusammensetzung des Zustroms Roh-IPDA II aus der ersten Vakuumdestillationskolonne in die zweite Vakuumdestillationskolonne die folgende Zusammensetzung aufweist:
| | |
|---|---|
| IPDA | 90-100 Gew.-% |
| Schwersieder | 0-10 Gew.-% |
| Rest-Ammoniak | 10-500 ppm. |

5. Verfahren nach mindestens einem der vorherigen Ansprüche, **dadurch gekennzeichnet, dass** die zweite Vakuumdestillationskolonne K II die folgenden Parameter aufweist:
| | |
|---|---|
| Druck | 10-200 mbar |
| Kopftemperatur | 80-200°C |
| Theoretische Stufen | 5-50. |

6. Verfahren nach mindestens einem der vorherigen Ansprüche, **dadurch gekennzeichnet, dass** der eingesetzte Partialkondensator am Kopf der zweiten Vakuumdestillationskolonne K II die folgenden Parameter aufweist:
| | |
|---|---|
| Druck | 10-200 mbar |
| Temperatur | 80-200°C. |

7. Verfahren nach Anspruch 1, **dadurch gekennzeichnet, dass** das Roh-Isophorondiamin I im Allgemeinen die folgende Zusammensetzung aufweist:
| | |
|---|---|
| IPDA | 75-100 Gew.-% |
| Wasser | 0-15 Gew.-% |
| Leichtsieder | 0-6 Gew.-% |
| Schwersieder | 0-6 Gew.-% |
| Rest-Ammoniak | 10-1000 ppm, |
und
dass die erste Vakuumdestillationskolonne K I die folgenden Parameter aufweist:
| | |
|---|---|
| Temperatur | 40-120°C |
| Druck | 10-200 mbar |
| Theoretische Stufen | 10-80, |
und
dass die Zusammensetzung des Zustroms Roh-IPDA II aus der ersten Vakuumdestillationskolonne in die zweite Vakuumdestillationskolonne die folgende Zusammensetzung aufweist:
| | |
|---|---|
| IPDA | 90-100 Gew.-% |
| Schwersieder | 0-10 Gew.-% |
| Rest-Ammoniak | 10-500 ppm, |
und
dass die zweite Vakuumdestillationskolonne K II die folgenden Parameter aufweist:
| | |
|---|---|
| Druck | 10-200 mbar |
| Kopftemperatur | 80-200°C |
| Theoretische Stufen | 5-50, |
und
dass der eingesetzte Partialkondensator am Kopf der zweiten Vakuumdestillationskolonne K II die folgenden Parameter aufweist:
| | |
|---|---|
| Druck | 10-200 mbar |
| Temperatur | 80-200°C. |

8. Verfahren nach mindestens einem der vorherigen Ansprüche, **dadurch gekennzeichnet, dass** die Reinheit des Rein-Isophorondiamins mindestens 98 Gew.-% beträgt und der Rest-Ammoniak-Gehalt im Rein-IPDA kleiner 50 ppm beträgt.

## Claims

1. Process for fine purification of isophoronediamine from the production of isophoronediamine by aminating hydrogenation of isophorone nitrile in the presence of at least ammonia, hydrogen, a hydrogenation catalyst and optionally further additions and in the presence or absence of organic solvents to obtain a crude isophoronediamine I,
**characterized in that** the crude isophoronediamine I is subjected to a fine purification by means of two vacuum distillation columns, wherein
I. in the first vacuum distillation column K I low-boiling by-products still present are removed, and a crude IPDA II is transferred from the bottom of K I into the vacuum distillation column K II,
II. and in the second vacuum distillation column K II the isophoronediamine is obtained in pure form overhead and separated from the organic residues, with two condensers being mounted at the top of the vacuum distillation column,
wherein the first condenser is a partial condenser and the pure IPDA is removed therein,
and wherein the second condenser is a total condenser and the residual portion of the vapour stream from K II is completely condensed therein and recycled as return stream into the first vacuum distillation column K I.

2. Process according to Claim 1, **characterized in that** the crude isophoronediamine I generally has the following composition:
| | |
|---|---|
| IPDA | 75-100 wt% |
| Water | 0-15 wt% |
| Low boilers | 0-6 wt% |
| High boilers | 0-6 wt% |
| Residual ammonia | 10-1000 ppm. |

3. Process according to at least one of the preceding claims, **characterized in that** the first vacuum distillation column K I has the following parameters:
| | |
|---|---|
| Temperature | 40-120°C |
| Pressure | 10-200 mbar |
| Theoretical plates | 10-80. |

4. Process according to at least one of the preceding claims, **characterized in that** the composition of the feed stream crude IPDA II from the first vacuum distillation column into the second vacuum distillation column has the following composition:
| | |
|---|---|
| IPDA | 90-100 wt% |
| High boilers | 0-10 wt% |
| Residual ammonia | 10-500 ppm. |

5. Process according to at least one of the preceding claims, **characterized in that** the second vacuum distillation column K II has the following parameters:
| | |
|---|---|
| Pressure | 10-200 mbar |
| Tops temperature | 80-200°C |
| Theoretical plates | 5-50. |

6. Process according to at least one of the preceding claims, **characterized in that** the partial condenser used at the top of the second vacuum distillation column K II has the following parameters:
| | |
|---|---|
| Pressure | 10-200 mbar |
| Temperature | 80-200°C. |

7. Process according to Claim 1, **characterized in that** the crude isophoronediamine I generally has the following composition:
| | |
|---|---|
| IPDA | 75-100 wt% |
| Water | 0-15 wt% |
| Low boilers | 0-6 wt% |
| High boilers | 0-6 wt% |
| Residual ammonia | 10-1000 ppm, |
and
**in that** the first vacuum distillation column K I has the following parameters:
| | |
|---|---|
| Temperature | 40-120°C |
| Pressure | 10-200 mbar |
| Theoretical plates | 10-80 |
and
**in that** the composition of the feed stream crude IPDA II from the first vacuum distillation column into the second vacuum distillation column has the following composition:
| | |
|---|---|
| IPDA | 90-100 wt% |
| High boilers | 0-10 wt% |
| Residual ammonia | 10-500 ppm, |
and
**in that** the second vacuum distillation column K II has the following parameters:
| | |
|---|---|
| Pressure | 10-200 mbar |
| Tops temperature | 80-200°C |
| Theoretical plates | 5-50 |
and
**in that** the partial condenser used at the top of the second vacuum distillation column K II has the following parameters:
| | |
|---|---|
| Pressure | 10-200 mbar |
| Temperature | 80-200°C. |

8. Process according to at least one of the preceding claims, **characterized in that** the purity of the pure isophoronediamine is at least 98% by weight and the residual ammonia content in the pure IPDA is less than 50 ppm.

## Revendications

1. Procédé de purification fine d'isophorone-diamine issue de la fabrication d'isophorone-diamine par hydrogénation aminante d'isophorone-nitrile en la présence au moins d'ammoniac, d'hydrogène, d'un catalyseur d'hydrogénation et éventuellement d'autres additifs et en la présence ou en l'absence de solvants organiques, une isophorone-diamine brute I étant obtenue, **caractérisé en ce que** l'isophorone-diamine brute I est soumise à une purification fine par deux colonnes de distillation sous vide,
I. dans la première colonne de distillation sous vide KI, la séparation de produits secondaires de point d'ébullition plus faible encore contenus ayant lieu, et une IPDA brute II étant transférée depuis le fond de KI dans la colonne de distillation sous vide KII,
II. et, dans la deuxième colonne de distillation sous vide KII, l'isophorone-diamine étant obtenue sous forme pure par la tête et séparée des résidus organiques, deux condensateurs étant disposés à la tête de la colonne de distillation sous vide,
le premier condensateur étant un condensateur partiel dans lequel l'IPDA pure est séparée,
et le deuxième condensateur étant un condensateur total dans lequel la partie restante du courant de vapeur de KII est complètement condensée et recyclée dans la première colonne de distillation sous vide KI en tant que courant de reflux.

2. Procédé selon la revendication 1, **caractérisé en ce que** l'isophorone-diamine brute I présente de manière générale la composition suivante :
IPDA : 75 à 100 % en poids,
eau : 0 à 15 % en poids,
composants de point d'ébullition faible : 0 à 6 % en poids,
composants de point d'ébullition élevé : 0 à 6 % en poids,
ammoniac résiduel : 10 à 1 000 ppm.

3. Procédé selon au moins l'une quelconque des revendications précédentes, **caractérisé en ce que** la première colonne de distillation sous vide KI présente les paramètres suivants :
température : 40 à 120 °C,
pression : 10 à 200 mbar,
étapes théoriques : 10 à 80.

4. Procédé selon au moins l'une quelconque des revendications précédentes, **caractérisé en ce que** la composition du courant d'alimentation d'IPDA brute II issu de la première colonne de distillation sous vide dans la deuxième colonne de distillation sous vide présente la composition suivante :
IPDA : 90 à 100 % en poids,
composants de point d'ébullition élevé : 0 à 10 % en poids,
ammoniac résiduel : 10 à 500 ppm.

5. Procédé selon au moins l'une quelconque des revendications précédentes, **caractérisé en ce que** la deuxième colonne de distillation sous vide KII présente les paramètres suivants :
pression : 10 à 200 mbar,
température de tête : 80 à 200 °C,
étapes théoriques : 5 à 50.

6. Procédé selon au moins l'une quelconque des revendications précédentes, **caractérisé en ce que** le condensateur partiel utilisé à la tête de la deuxième colonne de distillation sous vide KII présente les paramètres suivants :
pression : 10 à 200 mbar,
température : 80 à 200 °C.

7. Procédé selon la revendication 1, **caractérisé en ce que** l'isophorone-diamine brute I présente de manière générale la composition suivante :
IPDA : 75 à 100 % en poids,
eau : 0 à 15 % en poids,
composants de point d'ébullition faible : 0 à 6 % en poids,
composants de point d'ébullition élevé : 0 à 6 % en poids,
ammoniac résiduel : 10 à 1 000 ppm,
et
**en ce que** la première colonne de distillation sous vide KI présente les paramètres suivants :
température : 40 à 120 °C,
pression : 10 à 200 mbar,
étapes théoriques : 10 à 80,
et
**en ce que** la composition du courant d'alimentation d'IPDA brute II issu de la première colonne de distillation sous vide dans la deuxième colonne de distillation sous vide présente la composition suivante :
IPDA : 90 à 100 % en poids,
composants de point d'ébullition élevé : 0 à 10 % en poids,
ammoniac résiduel : 10 à 500 ppm,
et
**en ce que** la deuxième colonne de distillation sous vide KII présente les paramètres suivants :
pression : 10 à 200 mbar,
température de tête : 80 à 200 °C,
étapes théoriques : 5 à 50,
et
**en ce que** le condensateur partiel utilisé à la tête de la deuxième colonne de distillation sous vide KII présente les paramètres suivants :
pression : 10 à 200 mbar,
température : 80 à 200 °C.

8. Procédé selon au moins l'une quelconque des revendications précédentes, **caractérisé en ce que** la pureté de l'isophorone-diamine pure est d'au moins 98 % en poids et la teneur en ammoniac résiduelle dans l'IPDA pure est inférieure à 50 ppm.
